# EUROPEAN PATENT SPECIFICATION

(11) **EP 1 865 900 B1**
(45) Date of publication and mention of the grant of the patent: **17.07.2013**
(21) Application number: 06740540.7
(22) Date of filing: 30.03.2006
(51) Int. Cl.: A61F 13/15, A61F 13/505

(54) **DISPOSABLE ABSORBENT ARTICLES HAVING MULTIPLE REPLACEABLE ABSORBENT CORE COMPONENTS**
SAUGFÄHIGE EINWEGARTIKEL MIT MEHREREN AUSWECHSELBAREN SAUGFÄHIGEN KERNKOMPONENTEN
ARTICLES ABSORBANTS JETABLES COMPRENANT DE MULTIPLES COMPOSANTS DE NOYAU ABSORBANTS REMPLAÇABLES

(30) Priority: 06.04.2005 US 99791
(43) Date of publication of application: 19.12.2007
(73) Proprietor: The Procter & Gamble Company, Cincinnati, OH 45202 (US)
(72) Inventor: LAVON, Gary, Dean, Liberty Township, Ohio 45011 (US)
(74) Representative: McGregor, Judit Ester
(86) International application number: PCT/US2006/012630
(87) International publication number: WO 2006/108029

(56) References cited:
- EP-A- 1 495 740
- US-A- 5 906 602
- US-A1- 2002 091 368

## Description

### FIELD OF THE INVENTION

This invention relates to disposable absorbent articles having multiple replaceable absorbent core components.

### BACKGROUND OF THE INVENTION

A typical disposable absorbent article includes an absorbent core for receiving and holding bodily exudates discharged by the wearer of the article and is designed to be removed and discarded once the absorbent core becomes saturated with bodily discharges, such as urine. Other parts of the disposable absorbent article may still be usable, and except for being unitary with the absorbent core, these parts could continue to be used. In addition to the added cost and waste associated with discarding reusable materials, it is often inconvenient to remove and replace the entire disposable absorbent article when only a portion is saturated.

Disposable absorbent articles having removable absorbent inserts and thereby being potentially usable for more than a single saturation are known in the art. Examples of diapers having a replaceable absorben insert are described in EP 1495 740-A. However, because the absorbent insert typically is removable only from the interior, the over-garment must be removed from the wearer in order to remove the insert.

Accordingly, it would be desirable to provide a disposable absorbent article having a multi-piece absorbent core in which a primary absorbent core component is replaceable and forms a pocket inside which at least a portion of a replaceable secondary absorbent core component is contained, and in which at least the secondary absorbent core component is replaceable without having to remove the disposable absorbent article from the wearer.

### SUMMARY OF THE INVENTION

The present invention is a disposable absorbent article adapted to be worn about a lower torso of a human body, including a chassis forming a waist opening and a pair of leg openings and having opposed waist regions and a crotch region intermediate the waist regions, a primary replaceable absorbent core component disposed in at least the crotch region, and a secondary replaceable absorbent core component disposed at least partially inside a pocket formed by the primary replaceable absorbent core component and in capillary liquid communication with the primary replaceable absorbent core component.

### BRIEF DESCRIPTION OF THE DRAWINGS

In the accompanying drawing figures, like reference numerals identify like elements in various embodiments of the present invention. Elements shown in some figures have been omitted from other figures for the purpose of clarity.
FIG. 1 is a plan view of an exemplary disposable diaper of the present invention in its flat-out, uncontracted state, i.e., with all elastic induced contraction pulled out, with portions of the structure being cut away to more clearly show the construction of the diaper, and with the portion of the diaper that contacts the wearer facing the viewer;
FIG. 2 is an exploded perspective view depicting an exemplary disposable diaper, with the portion of the article that contacts the wearer facing upward;
FIG. 3 is a perspective, partially segmented illustration of an exemplary disposable diaper embodiment of the present invention;
FIG. 4 is a side view, showing in partial cross-section, the exemplary disposable diaper of FIG. 3;
FIG. 5 is a top plan view of an exemplary absorbent core useful in a disposable diaper according to the present invention;
FIG. 6 is an exploded perspective view depicting the relationship between the elements of an exemplary absorbent core of the present invention, with the portion of the core that faces the wearer facing upward;
FIG. 7 is a section view of an exemplary absorbent core similar to that shown in FIG. 3, taken along line 7-7, with the portion of the core that faces the wearer oriented upward;
FIG. 8 is a similar section view of another alternative exemplary absorbent core, with the portion of the core that faces the wearer oriented upward;
FIG. 9 is a similar section view of another alternative exemplary absorbent core, with the portion of the core that faces the wearer oriented upward;
FIG. 10 is a similar section view of another alternative exemplary absorbent core, with the portion of the core that faces the wearer oriented upward;
FIG. 11 is a similar section view of another alternative exemplary absorbent core, with the portion of the core that faces the wearer oriented upward;
FIG. 12 is a cross-section depiction of a structure providing access to replaceable absorbent core components.

### DETAILED DESCRIPTION OF THE INVENTION

In this description, the following terms have the following meanings:

Disposable absorbent article: A device that is placed against or in proximity to the body of a wearer to absorb and contain the various exudates discharged from the body. An exemplary embodiment of the present invention is a disposable diaper, but the present invention is applicable to a range of disposable absorbent articles such as incontinence briefs, incontinence undergarments, diaper holders and liners, training pants, pull-on diapers, and the like.

Absorbent core: An element of a disposable absorbent article containing a material or a combination of materials suitable for absorbing, distributing, and/or storing bodily liquids.

Attach, attached: Terms referring to elements being connected or united by fastening, adhering, bonding, etc. by any method suitable for the elements being attached together and their constituent materials, such as adhesive bonding, pressure bonding, thermal bonding, mechanical fastening, etc. Elements may be permanently attached together, in which case their detachment results in damage to one or both. Alternatively, elements may be releasably attached together such that their detachment can be accomplished without damage.

Chassis: A foundational constituent upon which the remainder of the structure of a disposable absorbent article is built up, *e.g*., in a disposable diaper, the structure that gives the disposable diaper the form of briefs or short pants when configured for wearing. A backsheet is typically a fundamental element of the chassis, usually in combination with a topsheet and/or additional structural elements.

Disposable diaper: An absorbent article generally worn by infants and incontinent persons about the lower torso so as to encircle the waist and the legs of the wearer and specifically adapted to receive and contain urinary and fecal waste.

Disposable: A term used to describe an absorbent article that is not designed to be laundered or otherwise restored or reused as an absorbent article after being removed from a wearer following saturation of its absorbent core, but is instead designed to be discarded after a single use. Note that, as described in this disclosure, a single use of a chassis may correspond to several replacements of one or more replaceable absorbent core components.

Disposed: A term referring to an element being attached and positioned in a particular place or position in a unitary structure with other elements.

Capillary liquid communication: The flow of a liquid from one absorbent element to another absorbent element by capillary transport. Also, a term used to describe a structural disposition of absorbent elements in which the flow of a liquid between them occurs through capillary transport of the liquid, requiring either the direct face-to-face contact of the absorbent elements with each other, the direct face-to-face contact of each of the absorbent elements with a hydrophilic intermediate layer providing capillary conduction of the liquid from one absorbent element to the other, or the protrusion of the fibers of a fibrous absorbent element through a porous and/or permeable intermediate layer into contact with the other absorbent element.

Interior and Exterior: Terms referring respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside". Also, when the absorbent article is oriented such that its interior faces upward, *e.g*., when it is laid out in preparation for setting the wearer on top of it, synonyms include "upper" and "lower" and "top" and "bottom", respectively.

Instructions: A term referring to an outline or delineation of steps to be performed. The descriptive nature of the instructions may be provided by text, by figures or diagrams, or by any other visual guide, such as by the sequential numbering of operations or of operable devices in correspondence with an effective sequence of steps of a method. The instructions may take any of several forms. For example, the instructions may be printed on the outside or the inside of an article of commerce, such as a package, itself, or may be in the form of a sheet or card contained inside or attached to a package. In some embodiments, the instructions may form part of the physical embodiment of a usable article as, for instance, when the instructions are printed on or affixed to a disposable diaper. In general, the instructions may be provided in any form in which the functional relationship between the instructions and the use of the article is clear to the user.

Lateral: A term referring to a direction running from a side edge to an opposing side edge of the article and generally perpendicular to the longitudinal direction. Directions within ±45° of the lateral direction are considered to be "lateral". The "width" of a component refers to its dimension in the lateral direction.

Longitudinal: A term referring to a direction running from a waist edge to an opposing waist edge of the article, generally parallel to the maximum linear dimension of the article and perpendicular to the lateral direction. Directions within ±45° of the longitudinal direction are considered to be "longitudinal". The "length" of a component refers to its dimension in the longitudinal direction.

Replaceable: A term used to describe the structural form of a component of a disposable absorbent article that can be replaced without damage to either the replaceable component or to any other part of the article, that is, that can be removed and for which a like component can be substituted in place of the removed component, without such damage. The structure must be adapted to allow for both the removal and the insertion of a component designated as replaceable. Thus, this term embraces an arrangement of structural elements in such a way as to make the designated component replaceable, in contrast to other arrangements of structural elements in ways that make other components non-removable and therefore non-replaceable.

Water-permeable and Water-impermeable: Terms referring to the penetrability of materials in the context of the intended usage of disposable absorbent articles. Specifically, the term "water-permeable" refers to a layer or a layered structure having pores, openings, and/or interconnected void spaces that permit liquid water to pass through its thickness in the absence of a forcing pressure. Conversely, the term "water-impermeable" refers to a layer or a layered structure through the thickness of which liquid water cannot pass in the absence of a forcing pressure. A layer or a layered structure that is water-impermeable according to this definition may be permeable to water vapor and thus "breathable". As is well known in the art, a common method for measuring the permeability to water of the materials typically used in absorbent articles is a hydrostatic pressure test, also called a hydrostatic head test or simply a "hydrohead" test. Suitable well known compendial methods for hydrohead testing are approved by INDA (formerly the International Nonwovens and Disposables Association, now The Association of the Nonwoven Fabrics Industry) and EDANA (European Disposables And Nonwovens Association). For the present purpose, the terms "permeable" and "impermeable" are considered to be interchangeable with "permeable" and "impermeable", respectively.

Body-facing: A term referring to the elements of the chassis or absorbent core that form the interior surface of the disposable absorbent article, such as the topsheet, the leg cuffs, and the side panels, *etc.,* when such elements are present.

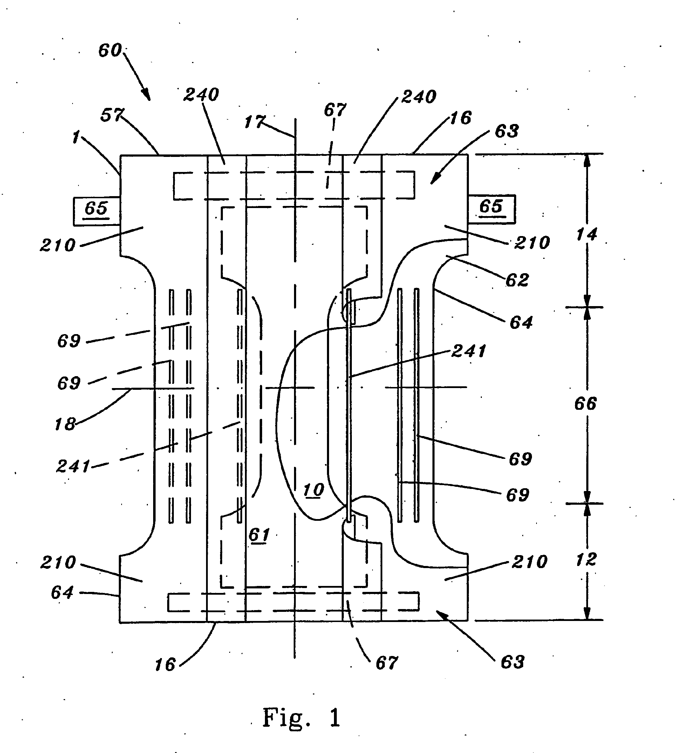

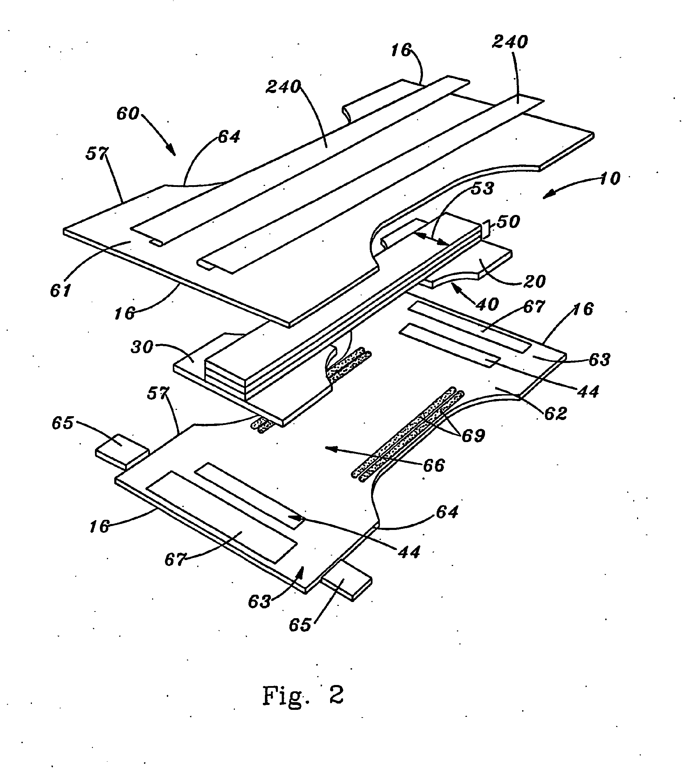

In the exemplary embodiments shown in FIGURE 1 and FIGURE 2, the disposable diaper **60** has a periphery **57** defined by the outer edges of the disposable diaper, with the longitudinal edges being designated **64** and the waist end edges being designated **16.** The disposable diaper additionally has a lateral axis which is designated **18** and a longitudinal axis which is designated **17.** The front waist region **12** and the back waist region **14** extend, respectively, from the waist end edges **16** toward the lateral axis **18** a distance from about 1/4 to about 1/3 the length of the disposable diaper. The crotch region **66** extends between the waist regions. An absorbent core **10** is disposed between a topsheet **61** and a backsheet **62.** The topsheet and the backsheet have length and width dimensions generally larger than those of the absorbent core and are attached together at or adjacent to portions of or the entirety of the periphery **57.**

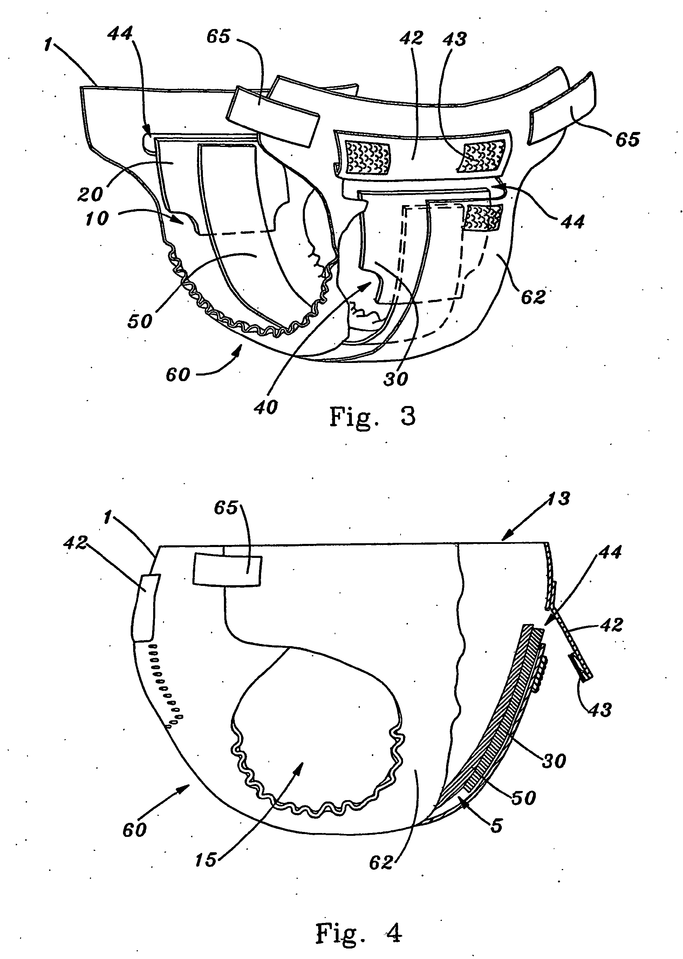

The multi-piece absorbent core **10** shown in FIGURE 2 and FIGURE 3 includes a primary absorbent core component **50** and two secondary absorbent core components, namely a front absorbent core component **20** and a back absorbent core component **30.** In some exemplary embodiments, the disposable absorbent article may include only one secondary absorbent core component or more than two secondary absorbent core components. Each secondary absorbent core component is replaceable. The primary absorbent core component may be non-removably disposed in the disposable absorbent article, such as by being secured, attached, affixed, and/or sandwiched to or in the chassis, as described in co-pending U.S. Patent Application No. 10/308,430 filed on 3 December 2002 and published as U.S. Patent Application Publication No. US 2003/0199844 A1 on 23 October 2003. However, preferably, the primary absorbent core component is replaceable and throughout the remainder of this disclosure is described as being so. In terms of liquid handling, the primary absorbent core component preferably has suitable liquid acquisition and distribution characteristics, while each secondary absorbent core component preferably has suitable liquid redistribution and storage characteristics.

An advantage provided by a multi-piece absorbent core is the capability to independently choose values for selected characteristics of the absorbent core components, such as liquid acquisition rates, liquid distribution rates, liquid storage capacities, interfacial liquid transfer rates and efficiencies, thicknesses, functionalities, and shapes or configurations. For example, in terms of liquid handling, the primary absorbent core component preferably has suitable liquid acquisition and distribution characteristics, while each secondary absorbent core component preferably has suitable liquid redistribution and storage characteristics. Two or more absorbent layers may form a primary replaceable absorbent core component, with the innermost layer having relatively greater liquid acquisition characteristics and the outer absorbent layer(s) having relatively greater liquid distribution characteristics. In this configuration, a liquid bodily discharge such as urine can be quickly acquired by the body-facing acquisition layer and can then be desorbed into the adjacent distribution layer(s), which can distribute it throughout the length of the primary replaceable absorbent core component. A secondary replaceable absorbent core component that is disposed in capillary liquid communication with the distribution layer(s) can then absorb the liquid and redistribute and store the liquid inside itself.

Thus, it becomes practical to assemble a disposable absorbent article of the present invention in a unique configuration suited for a particular use by placing a primary replaceable absorbent core component in position in at least the crotch region and placing a secondary replaceable absorbent core component in position such that it extends from one of the waist regions into the crotch region and is disposed in capillary liquid communication with the primary replaceable absorbent core component. If desired, another secondary replaceable absorbent core component may be placed in position such that it extends from the opposing waist region into the crotch region and is likewise disposed in capillary liquid communication with the primary replaceable absorbent core component.

For example, a primary replaceable absorbent core component having relatively fast liquid acquisition and distribution characteristics can be assembled together with a secondary replaceable absorbent core component having relatively high redistribution and storage characteristics, such as for use in keeping the skin of an infant dry at all times and especially during an extended period of sleep. Alternatively, a primary replaceable absorbent core component having relatively slow liquid acquisition and distribution characteristics can be assembled together with a secondary replaceable absorbent core component having relatively low redistribution and storage characteristics, such as for use in toilet training an older child for whom a feeling of wetness may facilitate recognition of an initial release of urine and/or a desire to use the toilet, rather than voiding into a disposable diaper. As another example, a primary replaceable absorbent core component having relatively fast liquid acquisition and distribution characteristics can be assembled together with a secondary replaceable absorbent core component having relatively low redistribution and storage characteristic, such as for use during brief absences from the home, when both keeping the wearer's skin dry and minimizing the bulk of the disposable diaper are important considerations.

Such assembly of the disposable absorbent article may be done at any time from manufacture to use, including in steps at different times so as to complete the assembly at the point of use. For example, a fully assembled disposable absorbent article may be provided in an article of commerce, such as a package. Alternatively, the chassis and one or both of the replaceable absorbent core components may be provided separately, either in the same article of commerce or separately. In any of these situations, instructions may be provided for assembling the disposable absorbent article as described in this disclosure.

An additional benefit resulting from the use of a multi-piece absorbent core is the capability to independently replace an absorbent core component to renew the functionality of that portion of the disposable absorbent article. For example, when a replaceable absorbent core component becomes saturated with bodily liquid, it may be removed and a fresh unsaturated like replacement absorbent core component may be inserted in its place to renew the associated portion of the absorptive capacity of the disposable absorbent article. In particular, as a secondary replaceable absorbent core component becomes saturated, it may become less effective at absorbing liquid from the primary replaceable absorbent core component. Consequently, the primary replaceable absorbent core component may become relatively more highly saturated and its ability to draw liquid away from the wearer's skin my be hindered. However, a replacement of the secondary replaceable absorbent core component renews the functionality of absorbing liquid from the primary replaceable absorbent core component. This replacement thereby leads to a reduction in the level of saturation of the primary replaceable absorbent core component and to a renewal of its ability to draw liquid away from the weaver's skin. Thus, the present invention makes it practical to renew the disposable diaper by way of a simple replacement of a replaceable absorbent core component.

The backsheet is compliant and readily conforms to the shape and contour of the wearer's body. The backsheet may include a water-impermeable material such as a thin plastic film in order to prevent the exudates absorbed and contained in the absorbent core from wetting external articles, such as bed sheets and clothing, which contact the disposable diaper. The backsheet may include a nonwoven layer disposed exteriorly of a film layer in order to provide a cloth-like appearance and feel on the exterior of the disposable diaper. The backsheet may be "breathable", i.e., water vapor-permeable, but water-impermeable, and thereby permit water vapor to escape from the absorbent core while preventing liquid exudates from escaping.

The topsheet is compliant, soft feeling, and non-irritating to the wearer's skin. At least a portion of the topsheet is water-permeable, thus permitting bodily liquids to readily penetrate through its thickness. A suitable topsheet can be manufactured from a wide range of materials such as woven and nonwoven materials, films, foams, and scrims. Suitable woven and nonwoven materials can include natural fibers, synthetic fibers, and/or combinations of natural and synthetic fibers. Preferably, the topsheet is made of a hydrophobic material to isolate the wearer's skin from liquids that have passed into the absorbent core.

It is also contemplated that a suitable absorbent core structure could be used without a topsheet to provide desirable results, such as comfort and absorbency, as well as simplicity in manufacturing and material cost savings. For example, the body-facing surface of the absorbent core could be made of a water-permeable, soft, compliant, non-irritating material, thereby making a separate topsheet unnecessary.

A portion of the backsheet and/or the topsheet may be subjected to mechanical stretching to form an extensible "zero strain" stretch laminate where such extensibility is desired, for example in elastic side panels. Suitable equipment and processes for such mechanical stretching and for the formation of such a zero strain stretch laminate are described in U.S. Patent 5,143,679 to Weber et al.*,* U.S. Patent 5,156,793 to Buell et al.*,* and U.S. Patent 5,167,897 to Weber et al.*.*

The chassis **1** of the disposable diaper may have an "open" configuration, as shown in FIGURE 1, in which it is adapted to be fastened together about the lower torso of a wearer by fastening means, such as the representative tape tab fasteners **65** that are disposed in a waistband region **63** for holding the disposable diaper on the wearer. The fastening means can be any of those well known in the art, such as mechanical fasteners, hook and loop fasteners, zippers, buttons, and the like. Alternatively, the chassis **1** may have a "pre-closed" configuration, such as that of a pull-on pant-type disposable diaper or training pants, in which it is preconfigured to be pulled on over the legs and lower torso of the wearer without any fastening steps. Starting from either configuration, the disposable diaper forms a waist opening **13** and leg openings **15** when configured for wearing, as shown in FIGURE 4.

The disposable diaper may have extensible side panels **210,** which preferably are elastically extensible, in order to maximize the ease of insertion and removal of the replaceable absorbent core components.

Leg elastic members **69** may be disposed adjacent to the periphery of the disposable diaper, preferably along each longitudinal edge **64** to form an elastically contractible leg cuff or side flap, so that the elastic members tend to draw and hold the disposable diaper against the legs of the wearer. The leg elastic members may extend along any portion of the length of the disposable diaper, up to and including the entire length.

A barrier leg cuff **240** including a barrier leg cuff elastic member **241** may be disposed adjacent to each longitudinal edge **64** or between the longitudinal edge and the longitudinal axis **17** of the disposable diaper. Suitable barrier leg cuff materials and structures are described in U.S. Patent 4,695,278 to Lawson, U.S. Patent 4,816,025 to Foreman, and U.S. Patent 4,808,178 to Aziz et al*.*

Waist elastic members **67** may be disposed in either the front, the back, or both of the waistband regions **63** of the disposable diaper. A suitable elasticized waist structure is described in U.S. Patent 4,515,595 to Kievit et al.*.*

The primary replaceable absorbent core component **50** may be generally rectilinear in shape, *i.e*., it may have a generally constant width **53** along its length, or it may vary in width along its length. At least adjacent to the lateral axis **18,** its width **53** preferably is suitable for comfortably fitting within the crotch area of the wearer.

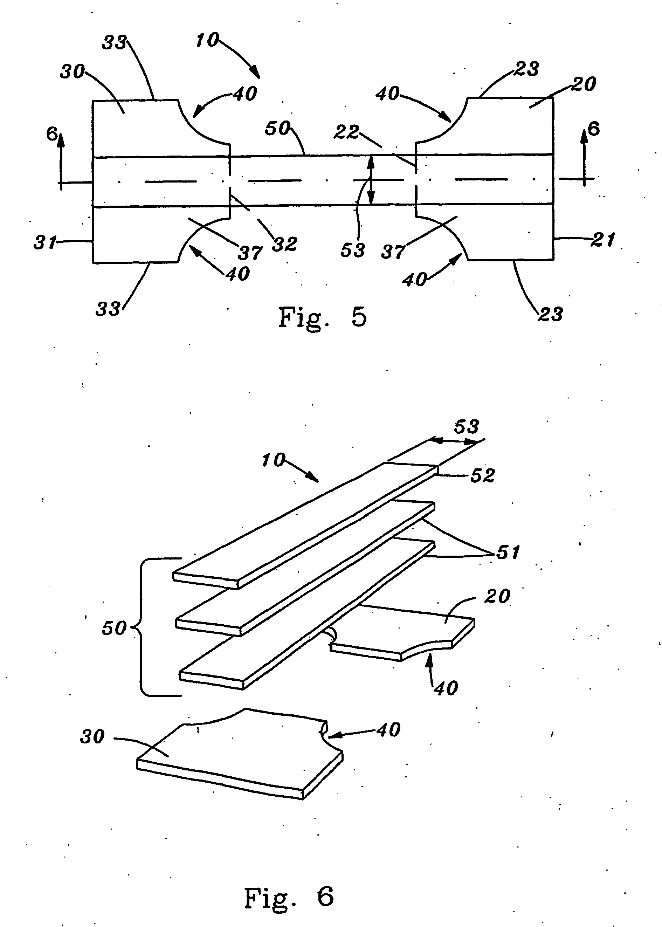

The primary replaceable absorbent core component is disposed in at least the crotch region **66.** In some embodiments, the primary replaceable absorbent core component may extend into one or both of the waist regions. For example, as shown in FIGURE 5, the primary replaceable absorbent core component may extend longitudinally from about the outer front end **21** of the front replaceable absorbent core component **20** to about the outer back end **31** of the back replaceable absorbent core component **30.** However, there is no need for the primary replaceable absorbent core component to extend any farther than is necessary to be disposed in capillary liquid communication with the front and/or back replaceable absorbent core components, such as by overlapping them.

The primary replaceable absorbent core component may be entirely free to "float" within the disposable diaper or may be releasably attached to another element over a part or the whole of any of its surfaces. For example, the primary replaceable absorbent core component may be releasably attached to the topsheet or to the backsheet or to both, and may be so attached in the crotch region. When such attachment is in the crotch region, the portions of the primary replaceable absorbent core component in the waist regions may remain unattached and thereby free to "float". Alternatively, only either the front portion or the back portion of the primary replaceable absorbent core component may be releasably attached and the longitudinally opposing portion may be allowed to float. Any such floating portion of the primary replaceable absorbent core component may form a pocket between the primary replaceable absorbent core component and the topsheet or the backsheet to receive and hold at least a portion of a secondary replaceable absorbent core component. For example, such a pocket **5** is shown between the primary replaceable absorbent core component **50** and the backsheet **62** in FIGURE 4.

As depicted in FIGURE 5, the front replaceable absorbent core component **20** has an outer front end **21,** an inner front end **22,** and a pair of sides **23.** Similarly, the back replaceable absorbent core component **30** has an outer back end **31,** an inner back end **32,** and a pair of sides **33.** The front replaceable absorbent core component generally lies in the front waist region with its inner front end **22** lying near or inside the crotch region and the back replaceable absorbent core component generally lies in the back waist region with its inner back end **32** lying near or inside the crotch region. Thus, in this exemplary embodiment, the front and back replaceable absorbent core components, together with the primary replaceable absorbent core component **50,** generally form an elongated hourglass shape.

It is preferred that a secondary replaceable absorbent core component extend into the crotch region no farther than the lowest point of the disposable diaper in the crotch of the wearer, so that it can be removed and replaced without the necessity of passing any portion of it, or the hand of the caregiver, or an insertion tool, through the wearer's crotch. Nevertheless, because the inner end can be shaped to fit comfortably into the crotch area of the wearer's body, the inner end can be optimally positioned for the absorption of liquid from the primary replaceable absorbent core component, which extends through the wearer's crotch and thus can serve to transport liquid from the front waist region to the back replaceable absorbent core component or from the back waist region to the front replaceable absorbent core component.

As shown in FIGURE 2, FIGURE 3, FIGURE 5, and FIGURE 6, the secondary replaceable absorbent core components may have tapering portions **37** defined by edge segments **40** extending between the sides **23, 33** and the inner ends such that the resulting widths of the inner ends **22** and **32** are narrower than those of the respective outer ends **21** and **31** and approach the width **53** of the primary replaceable absorbent core component near the lateral axis **18.** The edge segments of the tapering portion may be arcuate in contour, for example concavely arcuate as depicted in these figures, or may have another contour, such as a generally straight contour defining a trapezoidal shape or a convexly arcuate contour defining a rounded bullet shape (not shown).

When both front and back secondary replaceable absorbent core components are present, they preferably are disposed such that the inner front end **22** of the front replaceable absorbent core component 20 is spaced from the inner back end **32** of the back replaceable absorbent core component **30** as shown in FIGURE 2, FIGURE 3, and FIGURE 5. The back replaceable absorbent core component may be longer than the front replaceable absorbent core component or *vice versa.* A configuration in which the back replaceable absorbent core component is relatively longer lends itself to a relatively better fitting disposable diaper.

Like the primary replaceable absorbent core component, each secondary replaceable absorbent core component may be entirely free to "float" within the disposable diaper or may be releasably attached to another element over a part or the whole of any of its surfaces. For example, a secondary replaceable absorbent core component may be releasably attached to the topsheet or to the backsheet or to the primary replaceable absorbent core component.

Each of the primary and secondary replaceable absorbent core components may include multiple layers of absorbent material and each layer may have individual liquid handling characteristics, as well as an individual shape, width, length, and thickness. The number and placement of these absorbent layers may be varied to achieve desired characteristics, such as thinness, softness, flexibility, liquid acquisition rate, liquid distribution rate, and/or liquid storage capacity. For example, if the thickness of an individual layer of the primary replaceable absorbent core component would be detrimental to a wearer's comfort, this layer may not extend through the crotch region.

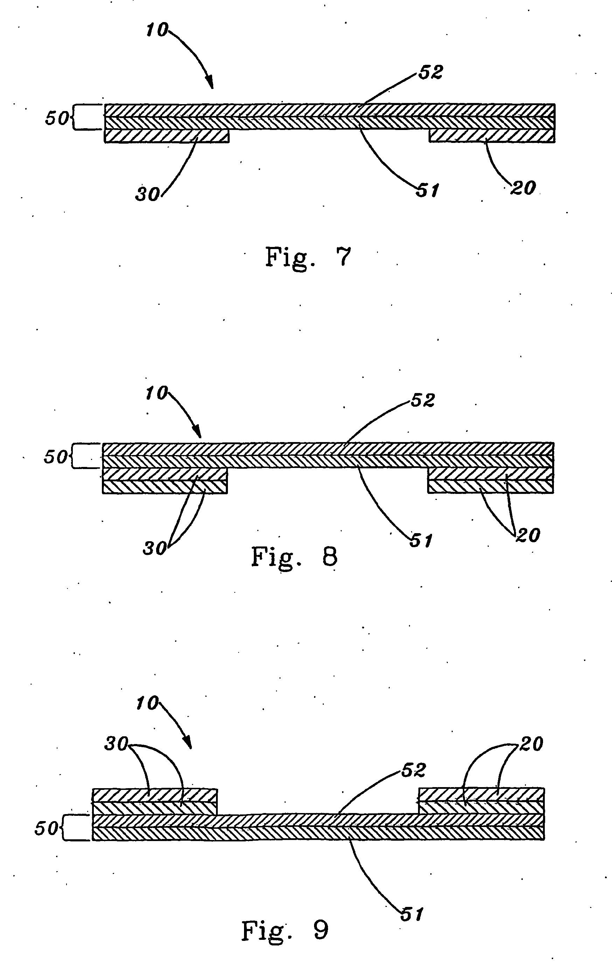

Several exemplary arrangements of the replaceable absorbent core components with respect to each other are shown in the figures. In the exemplary embodiment shown in cross section in FIGURE 7 and corresponding to the general top view of FIGURE 5, the primary replaceable absorbent core component **50** includes one upper absorbent layer 52 and one lower absorbent layer **51,** both of which interiorly overlap the singularly layered secondary replaceable absorbent core components **20** and **30.** In FIGURE 8, the absorbent layers **51** or **52** interiorly overlap dually layered secondary replaceable absorbent core components **20** and **30.** Thus, in an assembled disposable diaper of the exemplary configurations shown in FIGURE 7 and FIGURE 8, the secondary replaceable absorbent core components are disposed inside a pocket formed by and between the primary replaceable absorbent core component and the backsheet. In other embodiments, it may be beneficial to place the absorbent layers **51** or **52** so as to exteriorly overlap the secondary replaceable absorbent core components **20** and **30,** as shown in FIGURE 9. In an assembled disposable diaper of such alternative embodiments, the secondary replaceable absorbent core components are disposed inside a pocket formed by and between the primary replaceable absorbent core component and the topsheet.

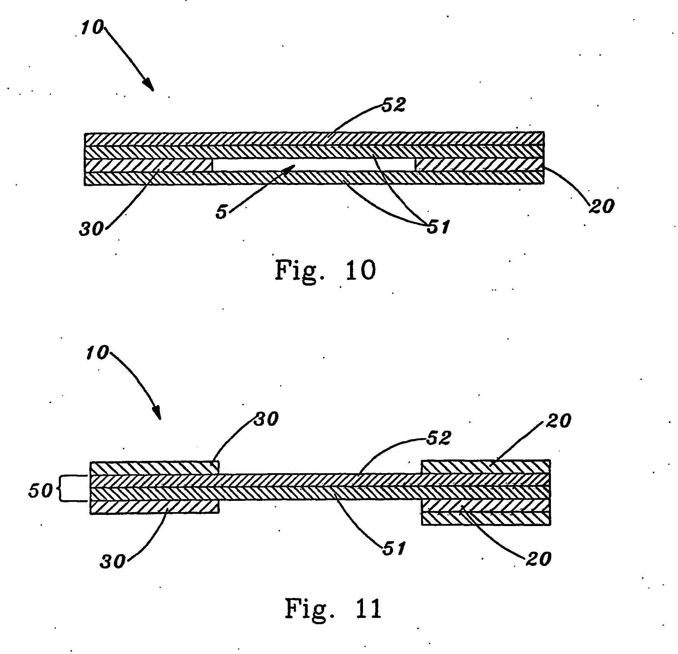

In FIGURE 10, the primary replaceable absorbent core component **50** has two absorbent layers **51,** one interiorly overlapping the secondary replaceable absorbent core components **20** and **30** and the other exteriorly overlapping the secondary replaceable absorbent core components, thereby sandwiching the secondary replaceable absorbent core components. Thus, in an assembled disposable diaper of this configuration, the secondary replaceable absorbent core components are disposed at least partially inside the pocket **5** formed by and between the layers **51** and **52** of the primary replaceable absorbent core component.

As another example, in FIGURE 11, each of the secondary replaceable absorbent core components **20** and **30** has two absorbent layers, one interiorly overlapping the primary replaceable absorbent core component **50** and one exteriorly overlapping the primary replaceable absorbent core component **50,** thereby sandwiching the ends of the primary replaceable absorbent core component. Thus, in an assembled disposable diaper of this configuration, one layer of each of the secondary replaceable absorbent core components is disposed inside a pocket formed by and between the primary replaceable absorbent core component and the topsheet and the other layer of each of the secondary replaceable absorbent core components is disposed inside a pocket formed by and between the primary replaceable absorbent core component and the backsheet.

Access to the replaceable absorbent core components may be provided at an aperture in the backsheet. For example, in the exemplary embodiment shown in FIGURE 2, FIGURE 3, and FIGURE 4, an aperture **44** in the back waist region provides access to the back replaceable absorbent core component **30** and to the primary replaceable absorbent core component **50.** A flap **42** may be provided to cover the aperture. When the disposable diaper is being worn, the flap may be secured over the aperture by a suitable fastener **43,** such as VELCRO or an adhesive strip (not shown). For example, FIGURE 4 shows a flap in the closed position over the aperture adjacent to the front panel **20** (shown in FIGURE 3). Preferably, the flap is sealed with releasable adhesive, thereby providing for liquid impermeability when closed, but allowing for multiple openings and closings.

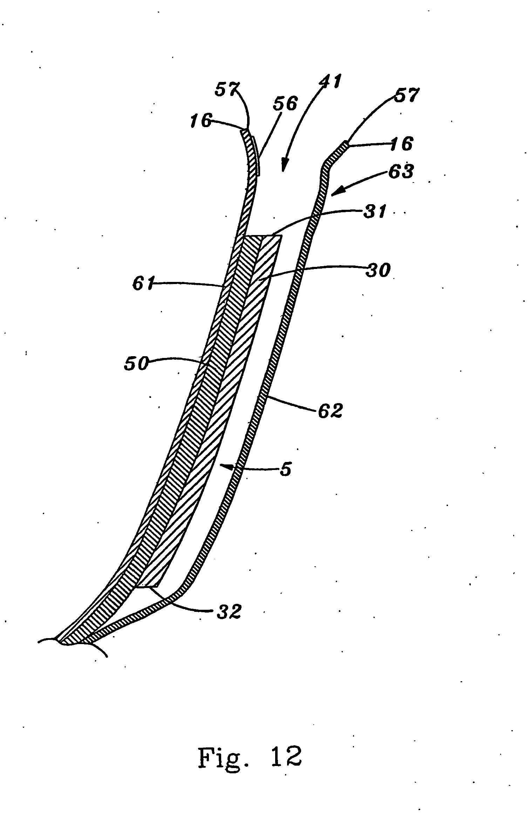

Access to the replaceable absorbent core components may be provided at a predetermined area of the periphery where the topsheet and the backsheet are separable to form an opening for the removal and/or insertion of the replacement absorbent core components. An example of such a structure is shown in cross-section in FIGURE 12, where the topsheet **61** and the backsheet **62** are separable along a predetermined area of the periphery **57,** in this embodiment along the waist end edge **16.** The opening **41** formed by the separation of the topsheet and the backsheet allows the removal and replacement of the replaceable absorbent core components and may be resealable when closed, for example with a suitable releasable and resealable adhesive **56** known in the art.

Suitable structures providing access to the replaceable absorbent core components are described in co-pending U.S. Patent Application No. 10/308,430 filed on 3 December 2002 in the name of LaVon et al. and published as U.S. Patent Application Publication No. US 2003/0199844 A1 on 23 October 2003.

Typical absorbent materials known in the art may be used for the replaceable absorbent core components, such as fibrous nonwoven materials, fibrous air-laid materials, fibrous wet-laid web materials, and/or combinations of fibrous materials having polymeric absorbent gelling materials dispersed upon or within the fibrous structure. Suitable absorbent materials may also be foam-based. Particularly suitable absorbent foams have been made from high internal phase emulsions. Exemplary materials and structures that may be utilized in the absorbent core structure of the present invention are described in U.S. Patent 5,531,728 to Lash, U.S. Patent 5,147,345 to Young et al.*,* U.S. Patent. 5,800,416 to Seger et al.*,* U.S. Patent 6,372,953 to Young et al.*,* U.S. Patent 5,260,345 to DesMarais et al.*,* U.S. Patent 5,268,224 to DesMarais et al.*,* U.S. Patent 5,387,207 to Dyer et al.*,* and U.S. Patent 5,563,179 to Stone et al*.* The absorbent core components may also include a combination of absorbent materials, for example, a combination of foam and wood pulp or other cellulosic fibers and/or particles or fibers of a polymeric absorbent gelling material.

Suitable materials and constructions for the replaceable absorbent core components are also described in the LaVon *et al.* '430 application. In particular, the primary replaceable absorbent core component of the present invention can be constructed similarly to the center section of the absorbent core in the LaVon *et al.* '430 application, with the exception that it must be made replaceable in the present invention. Likewise, the secondary replaceable absorbent core components of the present invention can be constructed similarly to the front and back panels of the LaVon *et al.* '430 application. Each absorbent core component may be formed into a packet in which the absorbent material is partially or wholly covered and/or enveloped by a water-permeable web material providing sufficient structural integrity for its handling during removal and replacement. The use of such a covering and/or enveloping material on the body-facing surface(s) of the absorbent core component(s) may obviate the need for a separate topsheet.

The relationship of the capillary absorption pressure of one absorbent element and the capillary desorption pressure of another absorbent element defines the liquid flow, liquid transfer, and capillary liquid communication characteristics of the disposable absorbent article. In particular, as is well-known in the art, liquid is absorbed from one layer to the next, from an acquisition layer to a storage layer, for example, if the absorbing layer's capillary absorption pressure exceeds the desorption pressure of the donating layer. This principle is that of a capillary cascade and suitable materials and ranges of properties are described in the Young *et al.* '345 patent and U.S. Patent 5,906,602 to Weber et al*.*

For example, the wearer-facing layer of the disposable absorbent article, *e.g*., a topsheet, is water-permeable and has particular capillary absorption and desorption pressures. The capillary desorption pressure of this topsheet is preferably less than the capillary absorption pressure of the primary replaceable absorbent core component and, specifically, less than the capillary absorption pressure of the uppermost absorbent layer of the primary replaceable absorbent core component, with which the topsheet will be in contact. In addition, it is preferred that the absorption pressure of a lower absorbent layer of the primary replaceable absorbent core component be greater than the capillary desorption pressure of the uppermost absorbent layer of the primary replaceable absorbent core component, such that liquid will be drawn toward this lower layer and away from the topsheet. Furthermore, it is preferable that the capillary absorption pressure of the secondary replaceable absorbent core component be greater than the capillary desorption pressure of the absorbent layer of the primary replaceable absorbent core component in contact with it, such that liquid will be drawn from the primary replaceable absorbent core component into the secondary replaceable absorbent core component.

The disposable absorbent articles of the present invention are preferably constructed such that liquid deposited in the article is quickly absorbed into the primary replaceable absorbent core component and then moved into the secondary replaceable absorbent core component. In order to minimize the overall bulk and to maximize the benefits of skin health and dryness, it is preferable that the primary replaceable absorbent core component have a liquid storage capacity that is low relative to the total capacity of the absorbent core, *i.e*., relative to the total of the summed capacities of the primary and secondary replaceable absorbent core components. Furthermore it is desirable to remove the majority of the liquid deposited in the article by removing the secondary replaceable absorbent core component for replacement with a fresh dry absorbent core component. Therefore, the liquid absorptive capacity of the secondary replaceable absorbent core component is preferably at least about 1.5 times, more preferably at least about 2 times, and most preferably at least about 4 times as great as the liquid absorptive capacity of the primary replaceable absorbent core component.

## Claims

1. A disposable absorbent article (60) adapted to be worn about a lower torso of a human body, comprising:
a chassis (1) forming a waist opening and a pair of leg openings and having longitudinally opposing front and back waist end edges (16), longitudinally opposing front and back waist regions (12, 14) adjacent to the respective waist end edges, and a crotch region (66) longitudinally intermediate of the waist regions;
a primary absorbent core component (50) disposed in at least the crotch region; and
a secondary absorbent core component (20, 30) disposed in capillary liquid communication with the primary absorbent core component,
characterise in that the primary absorbent core component is replaceable, the secondary absorbent core component is replaceable, and the secondary absorbent core component extends from one of the waist regions into and ends in the crotch region, and is disposed at least partially inside a pocket (5) formed by the primary absorbent core component.

2. The disposable absorbent article of Claim 1 wherein the primary absorbent core component extends from the front waist region through the crotch region into the back waist region.

3. The disposable absorbent article of any of the preceding claims wherein the secondary absorbent core component extends from the back waist region into and ends in the crotch region.

4. The disposable absorbent article of Claim 1 or Claim 2 wherein the secondary absorbent core component extends from the front waist region into and ends in the crotch region.

5. The disposable absorbent article of Claim 1 or Claim 2 comprising two of the secondary absorbent core components extending respectively from the back waist region and the front waist region into and ending in the crotch region.

6. The disposable absorbent article of any of Claims 1 through 4 whereby said pocket (5) is formed by and between the chassis and the primary absorbent core component and is adapted to receive and contain a portion of the secondary absorbent core component.

7. The disposable absorbent article of any of Claims I through 4. whereby said pocket (5) is formed by and between two layers (51, 52) of the primary absorbent core component and is adapted to receive and contain a portion of the secondary absorbent core component.

8. The disposable absorbent article of any of Claims 1 through 4 wherein the secondary absorbent core component has an outer end (21, 31), an inner end (22, 32), a pair of generally parallel sides (23, 33), and a tapering portion (37) defined by a pair of non-parallel edge segments (40) extending between the sides and the inner end such that the inner end is narrower than the outer end.

9. The disposable absorbent article of Claim 8 wherein at least one of the edge segments of the tapering portion is arcuate in contour.

10. The disposable absorbent article of Claim 9 comprising two of the secondary absorbent core components extending respectively from the back waist region and the front waist region into and ending in the crotch region, wherein the primary absorbent core component has a generally constant width (53) such that the secondary absorbent core components together with the primary absorbent core component form an elongated hourglass shape.

## Patentansprüche

1. Einweg-Absorptionsartikel (60), der dazu konzipiert ist, um den Unterleib eines menschlichen Körpers herum getragen zu werden, umfassend:
einen Rahmen (1), der eine Taillenöffnung und ein Paar Beinöffnungen bildet und in Längsrichtung gegenüberliegende vordere und hintere Taillenendränder (16), in Längsrichtung gegenüberliegende vordere und
hintere Taillenbereiche (12, 14), die an die jeweiligen Taillenendränder angrenzen, und einen in Längsrichtung zwischen den Taillenbereichen liegenden Schrittbereich (66) aufweist,
einen primären Absorptionskernbestandteil (50), der wenigstens in dem Schrittbereich angeordnet ist, und
einen sekundären Absorptionskernbestandteil (20, 30), der in kapillarem Flüssigkeitsaustausch mit dem primären Absorptionskernbestandteil angeordnet ist,
**dadurch gekennzeichnet, dass** der primäre Absorptionskernbestandteil austauschbar ist, der sekundäre Absorptionskernbestandteil austauschbar ist und sich der sekundäre Absorptionskernbestandteil von einem der Taillenbereiche in den Schrittbereich hinein erstreckt und darin endet und
wenigstens teilweise innerhalb einer Tasche (5) angeordnet ist, die durch den primären Absorptionskernbestandteil gebildet wird.

2. Einweg-Absorptionsartikel nach Anspruch 1, wobei sich der primäre Absorptionskernbestandteil vom vorderen Taillenbereich durch den Schrittbereich in den hinteren Taillenbereich hinein erstreckt.

3. Einweg-Absorptionsartikel nach einem der vorstehenden Ansprüche, wobei sich der sekundäre Absorptionskernbestandteil vom hinteren Taillenbereich in den Schrittbereich hinein erstreckt und darin endet.

4. Einweg-Absorptionsartikel nach Anspruch 1 oder Anspruch 2, wobei sich der sekundäre Absorptionskernbestandteil vom vorderen Taillenbereich in den Schrittbereich hinein erstreckt und darin endet.

5. Einweg-Absorptionsartikel nach Anspruch 1 oder Anspruch 2, umfassend zwei der sekundären Absorptionskernbestandteile, die sich jeweils vom hinteren Taillenbereich und vom vorderen Taillenbereich in den Schrittbereich hinein erstrecken und darin enden.

6. Einweg-Absorptionsartikel nach einem der Ansprüche 1 bis 4, wobei die Tasche (5) durch den Rahmen und den primären Absorptionskernbestandteil und dazwischen gebildet ist und so konzipiert ist, dass sie einen Abschnitt des sekundären Absorptionskernbestandteils aufnimmt und enthält.

7. Einweg-Absorptionsartikel nach einem der Ansprüche 1 bis 4, wobei die Tasche (5) durch zwei Schichten (51, 52) des primären Absorptionskernbestandteils und dazwischen gebildet ist und so konzipiert ist, dass sie einen Abschnitt des sekundären Absorptionskernbestandteils aufnimmt und enthält.

8. Einweg-Absorptionsartikel nach einem der Ansprüche 1 bis 4, wobei der sekundäre Absorptionskernbestandteil ein äußeres Ende (21, 31), ein inneres Ende (22, 32), ein Paar im Allgemeinen parallele Seiten (23, 33) und einen sich verjüngenden Abschnitt (37) aufweist, der durch ein Paar nicht parallele Randsegmente (40) bestimmt wird, die sich zwischen den Seiten und dem inneren Ende erstrecken, so dass das innere Ende schmaler ist als das äußere Ende.

9. Einweg-Absorptionsartikel nach Anspruch 8, wobei mindestens eines der Randsegmente des sich verjüngenden Abschnitts eine bogenförmige Kontur aufweist.

10. Einweg-Absorptionsartikel nach Anspruch 9, umfassend zwei der sekundären Absorptionskernbestandteile, die sich jeweils vom hinteren Taillenbereich und vom vorderen Taillenbereich in den Schrittbereich hinein erstrecken und darin enden, wobei der primäre Absorptionskernbestandteil eine im Allgemeinen konstante Breite (53) besitzt, so dass die sekundären Absorptionskernbestandteile zusammen mit dem primären Absorptionskernbestandteil eine längliche Sanduhrform bilden.

## Revendications

1. Article absorbant jetable (60) adapté pour être porté autour d'une partie inférieure du tronc d'un corps humain, comprenant :
un châssis (1) formant une ouverture de ceinture et une paire d'ouvertures de jambe et ayant des bords extrêmes de ceinture avant et arrière longitudinalement opposés (16), des régions de ceinture avant et arrière longitudinalement opposées (12, 14) adjacentes aux bords extrêmes de ceinture respectifs, et une région d'entrejambe (66) longitudinalement entre les régions de ceinture ;
un composant d'âme absorbante primaire (50) disposé dans au moins la région d'entrejambe ; et
un composant d'âme absorbante secondaire (20, 30) disposé en communication capillaire de liquide avec le composant d'âme absorbante primaire,
**caractérisé en ce que** le composant d'âme absorbante primaire est remplaçable, le composant d'âme absorbante secondaire est remplaçable, et le composant d'âme absorbante secondaire s'étend à partir d'une des régions de ceinture vers et se termine dans la région d'entrejambe, et est disposé au moins partiellement à l'intérieur d'une poche (5) formée par le composant d'âme absorbante primaire.

2. Article absorbant jetable selon la revendication 1, dans lequel le composant d'âme absorbante primaire s'étend de la région de ceinture avant à travers la région d'entrejambe vers la région de ceinture arrière.

3. Article absorbant jetable selon l'une quelconque des revendications précédentes, dans lequel le composant d'âme absorbante secondaire s'étend de la région de ceinture arrière vers et se termine dans la région d'entrejambe.

4. Article absorbant jetable selon la revendication 1 ou la revendication 2, dans lequel le composant d'âme absorbante secondaire s'étend de la région de ceinture avant vers et se termine dans la région d'entrejambe.

5. Article absorbant jetable selon la revendication 1 ou la revendication 2, comprenant deux des composants d'âme absorbante secondaire s'étendant respectivement de la région de ceinture arrière et de la région de ceinture avant vers et se terminant dans la région d'entrejambe.

6. Article absorbant jetable selon l'une quelconque des revendications 1 à 4, selon lequel ladite poche (5) est formée par et entre le châssis et le composant d'âme absorbante primaire et est adaptée pour recevoir et contenir une partie du composant d'âme absorbante secondaire.

7. Article absorbant jetable selon l'une quelconque des revendications 1 à 4, selon lequel ladite poche (5) est formée par et entre deux couches (51, 52) du composant d'âme absorbante primaire et est adaptée pour recevoir et contenir une partie du composant d'âme absorbante secondaire.

8. Article absorbant jetable selon l'une quelconque des revendications 1 à 4, dans lequel le composant d'âme absorbante secondaire a une extrémité externe (21, 31), une extrémité interne (22, 32), une paire de côtés généralement parallèles (23, 33), et une partie qui s'effile (37) définie par une paire de segments de bord non parallèles (40) s'étendant entre les côtés et l'extrémité interne de telle sorte que l'extrémité interne est plus étroite que l'extrémité externe.

9. Article absorbant jetable selon la revendication 8, dans lequel au moins l'un des segments de bord de la partie qui s'effile a un contour arqué.

10. Article absorbant jetable selon la revendication 9, comprenant deux des composants d'âme absorbante secondaire s'étendant respectivement à partir de la région de ceinture arrière et de la région de ceinture avant vers et se terminant dans la région d'entrejambe, dans lequel le composant d'âme absorbante primaire a une largeur généralement constante (53) de telle sorte que les composants d'âme absorbante secondaire conjointement avec le composant d'âme absorbante primaire forment une forme en sablier allongée.
